(19) Europäisches Patentamt
European Patent Office
Office européen des brevets

(11) Veröffentlichungsnummer: **0 115 266**
**A2**

(12) # EUROPÄISCHE PATENTANMELDUNG

(21) Anmeldenummer: 84100047.4

(22) Anmeldetag: 04.01.84

(51) Int. Cl.³: C 07 D 307/32
C 07 F 7/18

(30) Priorität: 28.01.83 US 461835

(43) Veröffentlichungstag der Anmeldung:
08.08.84 Patentblatt 84/32

(84) Benannte Vertragsstaaten:
AT BE CH DE FR GB IT LI NL

(71) Anmelder: F. HOFFMANN-LA ROCHE & CO. Aktiengesellschaft

CH-4002 Basel(CH)

(72) Erfinder: Baggiolini, Enrico Giuseppe
30 Evergreen Drive
North Caldwell, N.J.(US)

(72) Erfinder: Uskokovic, Milan Radoje
253 Highland Avenue
Upper Montclair N.J.(US)

(72) Erfinder: Wovkulich, Peter Michael
124 Rhoda Avenue
Nutley, N.J.(US)

(74) Vertreter: Mahé, Jean et al,
Postfach 3255 Grenzacherstrasse 124
CH-4002 Basel(CH)

(54) **Verfahren zur Herstellung von Cholecalciferolderivaten.**

(57) Verfahren zur Herstellung des 1α, 23,25-Trihydroxycholecalciferol-26-carbonsäure-23,26-lactons ausgehend von entsprechenden Dihydro-5-[2-(octahydro-7a-methyl-1H-inden-4-on-1-yl)-2-methyläthyl]-3-hydroxy-3-methyl-2(3H)-furanonderivaten und neue Zwischenprodukte.

EP 0 115 266 A2

Croydon Printing Company Ltd.

F.Hoffmann-La Roche & Co. Aktiengesellschaft, Basel, Schweiz

RAN 4212/37

# Verfahren zur Herstellung von Cholecalciferolderivaten

Die Erfindung betrifft ein Verfahren zur Herstellung des 1α,23,25-Trihydroxycholecalciferol-26-carbonsäure-23,26-lactons der Formel

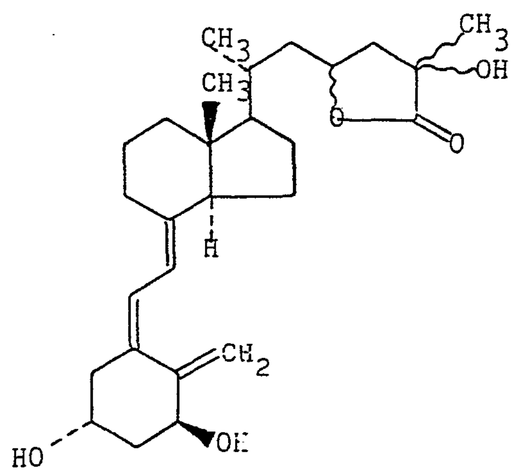

I

und neue Zwischenprodukte in dieser Herstellung. Dieses Verfahren ist dadurch gekennzeichnet, dass man

a) eine Verbindung der Formel

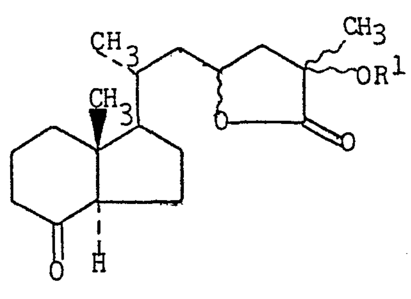

II

Mé/ 6.12.83

worin $R^1$ Wasserstoff, Tri-niederalkylsilyl, Aryl-diniederalkylsilyl, Diaryl-niederalkylsilyl oder Triarylsilyl ist,

mit einer Verbindung der Formel

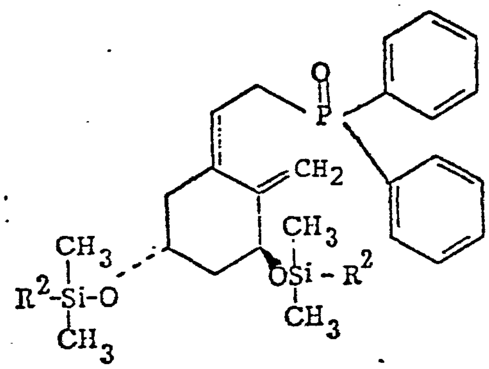

III

worin $R^2$ Niederalkyl oder Aryl ist,

zu einer Verbindung der Formel

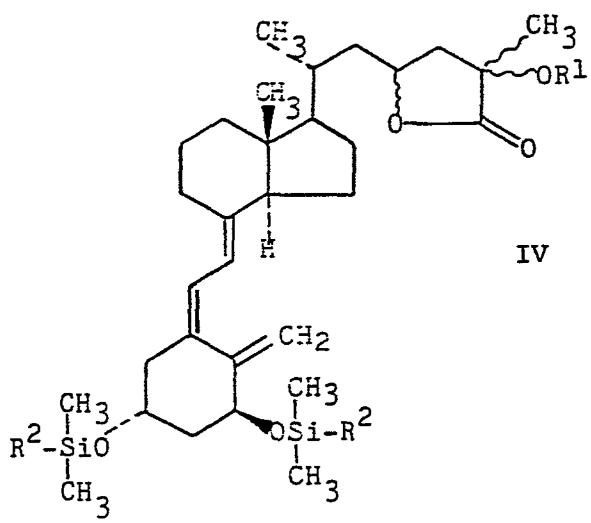

IV

worin $R^1$ und $R^2$ die obige Bedeutung haben,

umsetzt und

b) die Schutzgruppen entfernt.

Im Rahmen der vorliegenden Erfindung bezeichnet der Ausdruck "nieder" geradkettige oder verzweigte Gruppen mit bis zu 8 Kohlenstoffatomen. Beispiele von Niederalkylgruppen sind Methyl, Aethyl, n-Propyl, Isopropyl, t-Butyl, Hexyl, Heptyl und Octyl. Die $C_{3-6}$-Alkylengruppen sind geradkettig oder verzweigt. Beispiele davon sind Propylen, Butylen, Amylen und Hexylen. Der Ausdruck Aryl bezeichnet

eine aromatische Gruppe wie Phenyl und substituiertes Phenyl.

Anstelle eines der Diastereomeren der Formel II, z.B. des 3R,5S Diastereomeren [3R-[3β,5S,5β(R*)-[1R*-(1β,3aα,-7aα)]]]-Dihydro-5-[2-octahydro-7a-methyl-1H-inden-4-on-1-yl)-2-methyläthyl]-3-hydroxy-3-methyl-2(3H)-furanon kann man eines der folgenden Diastereomeren verwenden:

[3S-[3α,5R,5α(R*)-[1R*-(1β,3aα,4α,7aα)]]]-Dihydro-5-[2-octahydro-7a-methyl-1H-inden-4-on-1-yl)-2-methyläthyl]-3-hydroxy-3-methyl-2(3H)-furanon,

[3R-[3β-5R-5α(R*)-[1R*-(1β,4aα,4α,7aα)]]]-Dihydro-5-[2-(octahydro-7a-methyl-1H-inden-4-on-1-yl)-2-methyläthyl]-3-hydroxy-3-methyl-2(3H)-furanon oder

[3S-[3α,5S,5β(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon,

oder Gemische davon. Dabei werden die entsprechenden diastereomeren Verbindungen der Formel I oder Gemische davon erhalten.

Die Reaktionsstufe a) kann nach an sich bekannten Methoden, z.B. in Gegenwart einer Base, in einem Aetherlösungsmittel unter einer inerten Atmosphäre bei einer Temperatur im Bereich von etwa -80 bis -50°C durchgeführt werden. Beispiele von Basen sind Alkyllithiumverbindungen und Alkalimetalldialkylamide. Die Verbindung der Formel IV kann durch Elutionschromatographie auf Silicagel bereinigt werden.

Die Verfahrensstufe b) kann durch Behandlung der Verbindung der Formel IV mit einem Alkohol oder Wasser in Gegenwart einer Säure durchgeführt werden. Es kann dabei eine Mineralsäure, eine Niederalkancarbonsäure oder eine Sulfonsäure, vorzugsweise ein Ionenaustauscherharz in Form einer Suspension in einem niederen Alkanol, verwendet werden. Das Produkt der Formel I kann durch Filtrieren

des festen kationischen Ionenaustauscherharzes und Abdampfen von flüchtigen Produkten unter vermindertem Druck gereinigt werden.

Die Ausgangsmaterialien der Formel II können wie folgt hergestellt werden.

Ein Epoxid der Formel

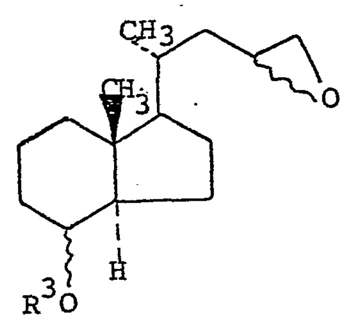

VI

wird mit einer Verbindung der Formel

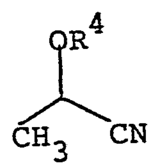

VII

worin $R^3$ Wasserstoff, Tri-niederalkylsilyl, Aryl-diniederalkylsilyl, Diaryl-niederalkylsilyl oder Triarylsilyl und $R^4$ Wasserstoff, Tri-niederalkylsilyl, Aryl-di-niederalkylsilyl, Diaryl-niederalkylsilyl, Triarylsilyl oder eine Gruppe der Formel

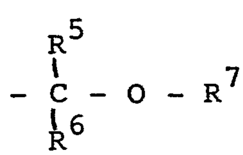

$R^5$ Wasserstoff oder Niederalkyl, $R^6$ und $R^7$ Niederalkyl oder Aryl oder $R^6$ und $R^7$ zusammen $C_{3-6}$-Alkylen sind,

in Gegenwart einer Base, in einem herkömmlichen Aetherlösungsmittel, unter einer inerten Atmosphäre und bei einer Temperatur im Bereich von etwa -80 bis +70°C umgesetzt. Man erhält dabei ein Gemisch von Verbindungen der

Formel

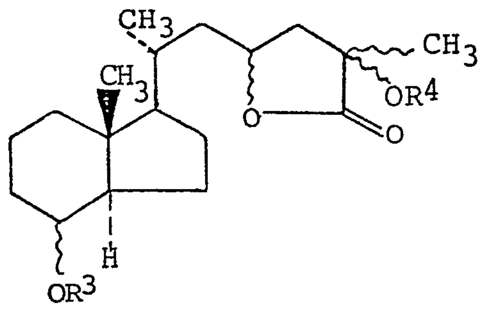

V

worin $R^3$ und $R^4$ die obige Bedeutung haben. Gewünschtenfalls können die so erhaltenen Diastereomere durch Elutionschromatographie auf Silicagel getrennt werden. Beispiele von Basen die im obigen Verfahren verwendet werden können sind Alkali- und Erdalkalimetalldialkyl- oder Disilylamide, wie Lithium-diisopropylamid, Kalium-bistrimethylsilylamid, Brommagnesium-diisopropylamid oder Kaliumhydrid.

Hydroxylschutzgruppen $R^3$ und $R^4$ können durch Behandlung mit einem Niederalkanol oder Wasser in Gegenwart einer Säure, z.B. einer Mineralsäure, wie Salzsäure oder Schwefelsäure, einer organischen Säure, wie Ameisensäure, Trifluoressigsäure oder Oxalsäure, oder einer Sulfonsäure, wie Methansulfonsäure oder Toluolsulfonsäure, abgespalten werden.

Die erhaltene Verbindung kann in eine Verbindung der Formel II, worin $R^1$ Wasserstoff ist, durch Behandlung mit einem Oxidationsmittel, z.B. einer Chrom enthaltenden Verbindung, wie Pyridiniumchlorchromat, 2,3-Bipyridiniumchlorchromat oder Pyridin-chromtrioxid, oder aktiviertem Dimethylsulfoxid, übergeführt werden.

Die erhaltene Verbindung der Formel II kann in eine Verbindung der Formel II, worin $R^1$ verschieden von Wasserstoff ist, durch Behandlung mit einem silylierenden Mittel in Gegenwart eines schwachen Amins übergeführt werden. Beispiele von Silylierungsmittel sind Trimethylchlorsilan, Triäthylchlorsilan, t-Butyldimethylchlorsilan und Chlordimethylphenylsilan. Als Base kann man Imidazol, 4-Dimethylaminopyridin oder Triazol verwenden. Ein bevorzugtes Silylierungsmittel ist Trimethylsilylimidazol, welches zweckmässig die freie Hydroxygruppe bei Raumtemperatur silyliert.

Die Ausgangsmaterialien der Formel VI können nach dem folgenden Verfahren hergestellt werden.

Eine Aethylidenverbindung der Formel

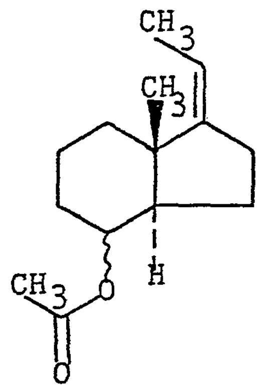

VIII

wird in einer En-Reaktion mit einem Niederalkyl-2-haloacrylat umgesetzt. Man erhält stereoselektiv in Bezug auf die γ-Position der Buttersäure-niederalkylester-Seitenkette eine Verbindung der Formel

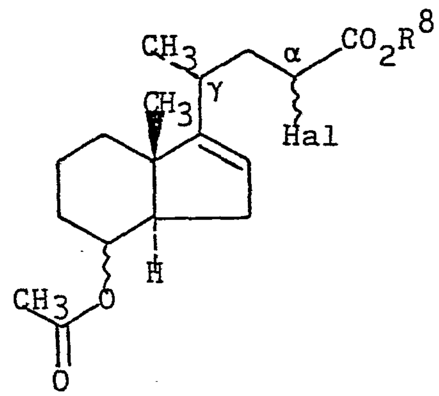

IX

worin $R^8$ Niederalkyl ist.

Vorzugsweise wird diese Reaktion in Gegenwart einer Lewis-Säure in einem inerten Lösungsmittel bei einer Temperatur im Bereich von etwa -20 bis 100°C, vorzugsweise von etwa

0°C bis Raumtemperatur durchgeführt. Beispiele von inerten Lösungsmittel sind Methylenchlorid, Tetrachlorkohlenstoff, Chloroform, aromatische Kohlenwasserstoffe, wie Benzol oder Toluol, und niederaliphatische Kohlenwasserstoffe, wie Hexan, Heptan oder Octan. Als Lewis-Säuren kann man Niederalkylaluminiumdihalogenide und Aluminiumtrihalogenide in einer schwachen Base, z.B. Aethylaluminiumdichlorid, Aluminiumtribromid oder Aluminiumchlorid in Pyridin, verwenden. Aethylaluminiumdichlorid ist bevorzugt.

Gewünschtenfalls kann das Gemisch der Stereoisomeren durch Elutionschromatographie aufgetrennt werden. Man erhält ein 1:6-Gemisch von R bzw. S Stereoisomeren an der α-Position.

Gewünschtenfalls kann ein R- oder S-Isomer oder ein Gemisch davon durch Aequilibrierung in ein 1:1-Gemisch der R und S Isomeren übergeführt werden. Die Aequilibrierung wird durch Umsetzung mit einer Verbindung der Formel IX mit Lithiumbromid durchgeführt. Die Temperatur der Reaktion ist nicht kritisch. Zweckmässig wird bei Raumtemperatur in einem polaren Lösungsmittel, wie Aceton, Dimethylformamid, Acetonitril, Dimethylsulfoxid oder Tetrahydrofuran, gearbeitet.

In der nächsten Stufe wird die Verbindung der Formel IX durch Reaktion mit einem Hydrid in einem inerten organischen Lösungsmittel zu einer Verbindung der Formel

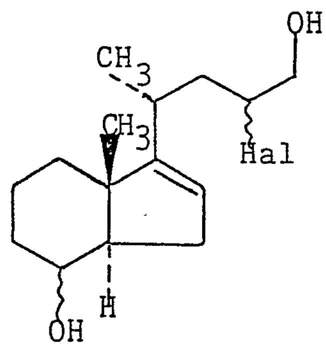

X

reduziert.

Beispiele von Hydriden sind Lithiumaluminiumhydrid oder vorzugsweise Diisobutylaluminiumhydrid. Beispiele von inerten organischen Lösungsmitteln sind niederaliphatische Kohlenwasserstoffe, wie Hexan, Heptan oder Octan, herkömmliche Aether, wie Diäthyläther oder Tetrahydrofuran, und aromatische Kohlenwasserstoffe, wie Benzol oder Toluol, oder Niederalkylhalogenide, wie Methylenchlorid, Chloroform oder Tetrachlorkohlenstoff. Die Reaktion wird bei einer Temperatur im Bereich von etwa -70 bis 80°C, vorzugsweise etwa 0°C bis Raumtemperatur, durchgeführt.

In der nächsten Stufe wird die Verbindung der Formel X mit einer Base in einem Lösungsmittel bei einer Temperatur im Bereich von etwa -70 bis 80°C, vorzugsweise etwa 0°C bis Raumtemperatur, zu einer Verbindung der Formel

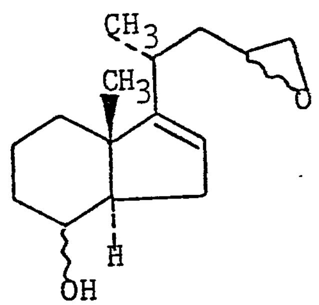

XI

umgesetzt.

Beispiele von Basen sind Kalium- oder Natrium-t-butoxid, Kaliumisopropoxid, Alkalimetallhydroxide, wie Natrium- oder Kaliumhydroxid, und Tri-niederalkylamine. Beispiele von Lösungsmitteln sind Niederalkanole, wie Methanol, Aethanol, t-Butanol oder Isopropanol, und herkömmliche Aether, wie Diäthyläther oder Tetrahydrofuran.

In der nächsten Stufe wird eine Verbindung der Formel XI mit Wasserstoff in Gegenwart eines Hydrierungskatalysators in einem inerten organischen Lösungsmittel bei einer Temperatur im Bereich von etwa 0 bis 80°C, vorzugsweise bei Raumtemperatur, zu einer Verbindung der Formel

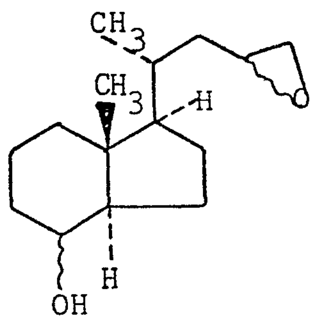

VIa

hydriert.

Beispiele von Hydrierungskatalysatoren sind 5% Platin auf Kohlenstoff, 5-10% Palladium auf Kohlenstoff, 5-10% Rhodium auf Kohlenstoff oder Platinoxid. Beispiele von Lösungsmittel sind niederaliphatische Kohlenwasserstoffe, wie Hexan, Heptan oder Octan, Niederalkanole, wie Methanol, Aethanol oder Propanol, herkömmliche Aether, wie Aethyläther oder Tetrahydrofuran, aromatische Kohlenwasserstoffe, wie Benzol oder Toluol, und Alkancarbonsäureniederalkylester, wie Aethylformiat oder Aethylacetat.

Gewünschtenfalls wird in der nächsten Stufe eine Verbindung der Formel VIa mit einem Silylierungsmittel unter einer inerten Atmosphäre bei einer Temperatur im Bereich von etwa 0-80°C, vorzugsweise bei Raumtemperatur, zu einer Verbindung der Formel VI, worin $R^3$ verschieden von Wasserstoff ist, umgesetzt. Beispiele von Silylierungsmittel sind Trimethylchlorsilan, Triäthylchlorsilan, t-Butyldimethylchlorsilan, Chlordimethylphenylsilan, Chlortriphenylsilan und vorzugsweise Trimethylsilylimidazol.

Die Verbindungen der Formel IV, worin $R^1$ Tri-niederalkylsilyl oder Wasserstoff und $R^2$ Niederalkyl sind, und die Verbindungen der Formeln II und V sind neu und als solche ebenfalls Gegenstand der vorliegenden Erfindung.

Beispiel 1

Einer Lösung von 1,18 g (0,0117 Mol) trockenem Diisopropylamin und 20 ml trockenem Tetrahydrofuran wurden bei 0°C unter einer inerten Atmosphäre 7,1 ml (0,0107 Mol) einer 1,5M Lösung von n-Butyllithium in Hexan zugesetzt. Dem auf -78°C abgekühlten Gemisch wurde eine Lösung von 1,137 g (0,0080 Mol) 2-(1-Aethoxyäthoxy)-propionitril in 25 ml trockenem Tetrahydrofuran tropfenweise zugesetzt. Das Gemisch wurde gerührt und dann wurde eine Lösung von 0,618 g (0,002 Mol) [1R-[1α(R*,S*),3aβ,4β,7aα]]octahydro-7a-methyl-1-(1-methyl-2-oxiranyläthyl)-1H-inden-4-ol-trimethylsilyläther in 15 ml trockenem Tetrahydrofuran tropfenweise zugesetzt. Das Eisbad wurde entfernt und das Gemisch wurde bei Raumtemperatur gerührt. Dann wurden 7 ml Wasser zugesetzt und das Gemisch wurde bei Rückflusstemperatur erhitzt. 30 ml 1,3M wässrige Weinsäure wurden zugesetzt und das Gemisch wurde bei Rückflusstemperatur erhitzt. Durch Zugabe einer zusätzlichen Portion Weinsäure wurde der pH auf 2 gebracht, das Gemisch wurde gerührt, dann wurden 100 ml Wasser zugesetzt und das Gemisch wurde mit 3 x 50 ml Methylenchlorid extrahiert. Die vereinigten Extrakte wurden mit Wasser bis zur Neutralität gewaschen, dann über wasserfreiem Natriumsulfat getrocknet. Das Gemisch wurde filtriert und das Lösungsmittel unter vermindertem Druck entfernt. Man erhielt ein Gemisch von [3R-[3β,5S,5β(R*)-[1R*(1β,3α,-4α,7aα)]]]Dihydro-5-[2-(octahydro-4-trimethylsilyloxy-7a-methyl-1H-inden-1-yl)-2-methyläthyl]-3-(1-äthoxyäthoxy)-3-methyl-2(3H)-furanon und [3S-[3α,5S,5β(R*)-[1R*-(1β,3aα,-4α,7aα)]]]-Dihydro-5-[2-(octahydro-4-trimethylsilyloxy-7a-methyl-1H-inden-1-yl)-2-methyläthyl]-3-(1-äthoxyäthoxy)-methyl-2(3H)-furanon, welches in der nächsten Stufe direkt verwendet wurde.

Beispiel 2

Das nach Beispiel 1 erhaltene rohe Gemisch wurde in 100 ml Methanol gelöst und mit 0,25 g Toluolsulfonsäuremonohydrat bei Raumtemperatur gerührt, dann wurde das Lösungsmittel unter vermindertem Druck entfernt. Dem Rückstand wurden 150 ml Wasser zugesetzt und das Gemisch wurde mit 3 x 50 ml Diäthyläther-Hexan extrahiert. Die vereinigten Extrakte wurden bis zur Neutralität mit einer Salzlauge gewaschen, dann über wasserfreiem Natriumsulfat getrocknet. Nach Filtrieren und Abdampfen des Lösungsmittels unter vermindertem Druck erhielt man 0,635 g Rückstand. Der Rückstand wurde auf Silicagel mit Aethylacetat/Hexan/Isopropanol als Eluierungsmittel chromatographiert. Man erhielt 0,247 g (40%) [3R-[3β,5S,5β(R*)-1R*-(1β,3aα,4α,7aα)]]Dihydro-5- [2-(octahydro-4-hydroxy-7a-methyl-1H-inden-1-yl)-2-methyläthyl]-3-hydroxy-3-methyl-2(3H)-furanon von Smp. 129,5-130,5°C nach Umkristallisation aus Aethylacetat; $[\alpha]_D^{25}$ + 3,54° (c, 0,4545, $CHCl_3$), und 0,189 g [3S-[3α,5S,5β(R*)-1R*-(1β,3aα,4α,7aα)]]-Dihydro-5-[2-(octahydro-4-hydroxy-7a-methyl-1H-inden-1-yl)-2-methyläthyl]-3-hydroxy-3-methyl-2(3H)-furanon, Smp. 190-191°C, $[\alpha]_D^{25}$ - 27,59° (c, 0,986, $CHCl_3$).

## Beispiel 3

Eine Lösung von 90 mg (0,290 mMol) [3R-[3β,5S,5β(R*)-1R*-(1β,3aα,4α,7aα)]]-Dihydro-5-[2-(octahydro-4-hydroxy-7a-methyl-1H-inden-1-yl)-2-methyläthyl]-3-hydroxy-3-methyl-2(3H)-furanon in 4 ml trockenem Methylenchlorid wurde bei Raumtemperatur mit 340 mg (1,162 mMol) 2,2'-Bipyridiniumchlorochromat versetzt. Dann wurden ein par Tropfen Isopropylalkohol zugesetzt und das erhaltene Gemisch wurde mit 50 ml Methylenchlorid verdünnt und filtriert. Nach Abdampfen des Lösungsmittels wurde der Rückstand in 100 ml Aethylacetat gelöst, mit 3 x 20 ml 1N Salzsäure und dann mit 3 x 30 ml Salzlauge extrahiert. Die organischen Phasen wurden vereinigt, getrocknet und eingedampft. Man erhielt 89 mg (quantitative Ausbeute) [3R-[3β,5S,5β(R*)-1R*-(1β,-3aα,4α,7aα)]]-Dihydro-5-[2-(octahydro-7a-methyl-1H-inden-4-on-1-yl)-2-methyläthyl]-3-hydroxy-3-methyl-2(3H)-furanon.

Beispiel 4

Eine Lösung von 89 mg (0,289 mMol) [3R-[3β,5S,5β(R*)-1R*-(1β,3aα,4α,7aα)]]-Dihydro-5-[2-(octahydro-7a-methyl-1H-inden-4-on-1-yl)-2-methyläthyl]-3-hydroxy-3-methyl-2(3H)-furanon in 5 ml trockenem Tetrahydrofuran wurde mit 200 mg (1,426 mMol) N-Trimethylsilylimidazol versetzt und das erhaltene Gemisch wurde unter Argon gerührt. 0,5 ml Wasser wurden zugesetzt und nach Rühren wurde das erhaltene Gemisch mit Aethylacetat extrahiert. Die vereinigten organischen Phasen wurden mit einer Salzlauge gewaschen, getrocknet und eingedampft. Der Rückstand wurde durch Filtrierung gereinigt, mit Hexan-Aethylacetat eluiert und man erhielt 93 mg (86% Ausbeute) [3R-[3β,5S,5β(R*)-1R*-(1β,3aα,4α,7aα)]]-Dihydro-5-[2-(octahydro-7a-methyl-1H-inden-4-on-1-yl)-2-methyläthyl]-3-trimethylsilyloxy-3-methyl-2(3H)-furanon.

Beispiel 5

Eine Lösung von 260 mg (0,446 mMol) [3S-(3α,5β,Z)]-2-[2-Methylen-3,5-bis-[(1,1-dimethyläthyl)dimethylsilyloxy]-cyclohexyliden]äthyldiphenylphosphinoxid in 6 ml trockenem Tetrahydrofuran wurde auf -78°C abgekühlt und tropfenweise unter Argon mit 0,250 ml (0,425 mMol) einer 1,7M-Lösung von n-Butyllithium in Hexan versetzt. Nach Rühren wurde eine Lösung von 94 mg (0,247 mMol) [3R-[3β,5S,5β,(R*)-1R*-(1β,3aα,4α,7aα)]]-Dihydro-5-[2-(octahydro-7a-methyl-1H-inden-4-on-1-yl)-2-methyläthyl]-3-trimethylsilyloxy-3-methyl-2(3H)-furanon in 2 ml trockenem Tetrahydrofuran langsam versetzt und das erhaltene Gemisch wurde bei -78°C gerührt. Es wurde dann mit 2 ml eines 1:1-Gemisches von 1N Natriumbicarbonat und 1N Kaliumnatriumtartrat versetzt, auf Raumtemperatur erwärmen gelassen, mit Wasser verdünnt und mit 3 x 60 ml Aethylacetat extrahiert. Die vereinigten organischen Extrakte wurden mit Salzlauge gewaschen, getrocknet und eingedampft. Der Rückstand wurde durch Chromatographie auf Kieselerde mit Hexan/Aethylacetat als Eluierungsmittel chromatographiert. Man erhielt 160 mg (1α,3β,-5Z,7E,23S,25R)-1,3-Bis[dimethyl-(1,1-dimethyläthyl)silyloxy]-23-hydroxy-25-trimethylsilyloxy-9,10-secocholesta-5,7,10(19)-trien-26-carbonsäure-γ-lacton in Form eines trüben farblosen Oels.

## Beispiel 6

Die 160 mg Lacton des Beispiels 5 wurden in 6 ml trockenem Methanol gelöst, mit 1,6 g eines kationenaustauschenden Harzes behandelt und gerührt. Nach Filtrieren und Waschen des Harzes mit 20 ml Methanol wurde das Lösungsmittel im Vakuum abgedampft und der Rückstand wurde in 80 ml Aethylacetat gelöst und mit 2 x 20 ml 2N Natriumbicarbonatlösung gefolgt durch 3 x 20 ml Salzlauge gewaschen. Der nach Abdampfen des Lösungsmittels erhaltene Rückstand wurde durch Chromatographie auf Silicagel mit Aethylacetat als Eluierungsmittel gereinigt. Man erhielt 101 mg (92%) reines (1α,3β,5Z,7E,23S,25R)-1,3,23,25-Tetrahydroxy-9,10-secocholesta-5,7,10(19)-trien-26-carbonsäure-γ-lacton in Form eines weissen amorphen Pulvers.

## Beispiel 7

Nach dem Verfahren von Beispiel 3 wurden

a) [3S-[3α,5S,5β(R*)-[1R*-(1β,3αa,4α,7aα)]]]-Dihydro-5-[2-(octahydro-4-hydroxy-7a-methyl-1H-inden-1-yl)-2-methyläthyl]-3-hydroxy-3-methyl-2(3H)-furanon

in

[3S-[3α,5S,5β(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon;

b) [3S-[3α,5R,5α(R*)-[1R*-(1β,3aα,4α,7aα)]]]-Dihydro-5-[2-(octahydro-4-hydroxy-7a-methyl-1H-inden-1-yl)-2-methyl-

äthyl]-3-hydroxy-3-methyl-2(3H)-furanon

in

[3S-[3α,5R,5α(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon; und

c) [3R-[3β,5R,5α(R*)-[1R*-(1β,3aα,4α,7aα)]]]-Dihydro-5-[2-(octahydro-4-hydroxy-7a-methyl-1H-inden-1-yl)-2-methyläthyl]-3-hydroxy-3-methyl-2(3H)-furanon

in

[3R-[3β,5R,5α(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon.

übergeführt.

## Beispiel 8

Nach dem Verfahren von Beispiel 4 wurden

a) [3S,[3α,5S,5β(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon

in

[3S-[3α,5S,5β(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon;

b) [3S-[3α,5R,5α(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon

in

[3S-[3α,5R,5α(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon; und

c) [3R-[3β,5R,5α(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon

in

[3R-[3β,5R,5α(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon übergeführt.

## Beispiel 9

Nach dem Verfahren von Beispiel 5 wurden

a) [3S-[3α,5R,5α(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon

in

(1α,3β,5Z,7E,23R,25S)-1,3-Bis(dimethyl-(1,1-dimethyläthyl)silyloxy]-23-hydroxy-25-trimethylsilyloxy-9,10-seco cholesta-5,7,10(19)-trien-26-carbonsäure-γ-lacton;

b) [3R-[3β,5R,5α(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon

in

(1α,3β,5Z,7E,23R,25R)-1,3-Bis[dimethyl-(1,1-dimethyl-

äthyl)silyloxy]-23-hydroxy-25-trimethylsilyloxy-9,10-secocholesta-5,7,10(19)-trien-26-carbonsäure-γ-lacton;

und

c) das [3S-[3α,5S,5β(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon

in

(1α,3β,5Z,7E,23S,25S)-1,3-Bis[dimethyl-(1,1-dimethyläthyl)silyloxy]-23-hydroxy-25-trimethylsilyloxy-9,10-secocholesta-5,7,10(19)-trien-26-carbonsäure-γ-lacton übergeführt.

## Beispiel 10

Nach dem Verfahren von Beispiel 6 wurden

a) (1α,3β,5Z,7E,23R,25R)-1,3-Bis[dimethyl-(1,1-dimethyläthyl)silyloxy]-23-hydroxy-25-trimethylsilyloxy-9,10-secocholesta-5,7,10(19)-trien-26-carbonsäure-γ-lacton

in

(1α,3β,5Z,7E,23R,25R)-1,3,23,25-Tetrahydroxy-9,10-secocholesta-5,7,10(19)-trien-26-carbonsäure-γ-lacton;

b) (1α,3β,5Z,7E,23R,25S)-1,3-Bis[dimethyl-(1,1-dimethyläthyl)silyloxy]-23-hydroxy-25-trimethylsilyloxy-9,10-secocholesta-5,7,10(19)-trien-26-carbonsäure-γ-lacton

in

(1α,3β,5Z,7E,23R,25S)-1,3,23,25-Tetrahydroxy-9,10-secocholesta-5,7,10(19)-trien-26-carbonsäure-γ-lacton; und

c) (1α,3β,5Z,7E,23S,25S)-1,3-Bis[dimethyl-(1,1-dimethyläthyl)silyloxy]-23-hydroxy-25-trimethylsilyloxy-9,10-secocholesta-5,7,10(19)-trien-26-carbonsäure-γ-lacton

in

(1α,3β,5Z,7E,23S,25S)-1,3,23,25-Tetrahydroxy-9,10-secocholesta-5,7,10(19)-trien-26-carbonsäure-γ-lacton übergeführt.

## Beispiel 11

Nach dem Verfahren von Beispiel 1 wurde

[1R-[1α(R*,R*),3aβ,4β,7aα]]-Octahydro-7-methyl-1-(1-methyl-2-oxiranyläthyl)-1H-inden-4-ol-trimethylsilyläther

in

[3R-[3β,5R,5α(R*)-[1R*-(1β,3aα,4α,7aα)]]]-Dihydro-5-[2-(octahydro-4-trimethylsilyloxy-7a-methyl-1H-inden-1-yl)-2-methyläthyl]-3-(1-äthoxyäthoxy)-3-methyl-2(3H)-furanon

und

[3S-[3α,5R,5α(R*)-[1R*-(1β,3aα,4α,7aα)]]]-Dihydro-5-[2-(octahydro-4-trimethylsilyloxy-7a-methyl-1H-inden-1-yl)-2-methyläthyl]-3-(1-äthoxyäthoxy)-3-methyl-2(3H)-furanon übergeführt.

## Beispiel 12

Nach dem Verfahren von Beispiel 2 wurden

[3R-[3β,5R,5α(R*)-[1R*-(1β,3aα,4α,7aα)]]]-Dihydro-5-[2-(octahydro-4-trimethylsilyloxy-7a-methyl-1H-inden-1-yl)-2-methyläthyl]-3-(1-äthoxyäthoxy)-3-methyl-2(3H)-furanon und

[3S-[3α,5R,5α(R*)-[1R*-(1β,3aα,4α,7aα)]]]-Dihydro-5-[2-(octahydro-4-trimethylsilyloxy-7a-methyl-1H-inden-1-yl)-2-methyläthyl]-3-(1-äthoxyäthoxy)-3-methyl-2(3H)-furanon

in

[3R-[3β,5R,5α,(R*)-[1R*-(1β,3aα,4α,7aα)]]]-Dihydro-5-[2-(octahydro-4-hydroxy-7a-methyl-1H-inden-1-yl)-2-methyläthyl]-3-hydroxy-3-methyl-2(3H)-furanon, Smp. 166-167°C, $[\alpha]_D^{25}$ = + 58,39° (c, 0,3819, $CHCl_3$) bzw.

[3S-[3α,5R,5α(R*)-[1R*-(1β,3aα,4α,7aα)]]]-Dihydro-5-[2-(octahydro-4-hydroxy-7a-methyl-1H-inden-1-yl)-2-methyläthyl]-3-hydroxy-3-methyl-2(3H)-furanon, Smp. 151-152°C, $[\alpha]_D^{25}$ = + 33,47°, (c, 0,98, $CHCl_3$) übergeführt.

## Patentansprüche

1. Verfahren zur Herstellung des 1α,23,25-Trihydroxy-cholecalciferol-26-carbonsäure-23,26-lactons der Formel

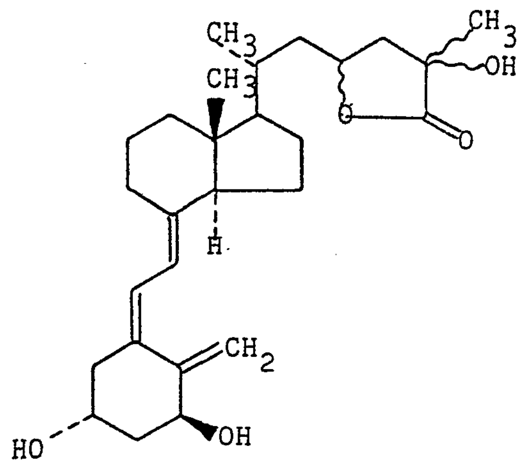

I

dadurch gekennzeichnet, dass man

a) eine Verbindung der Formel

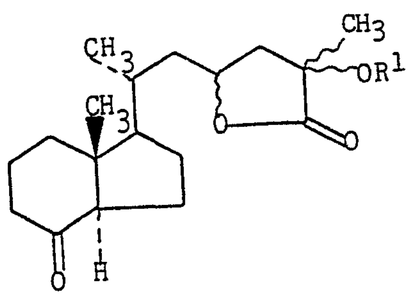

II

worin $R^1$ Wasserstoff, Tri-niederalkylsilyl, Aryl-diniederalkylsilyl, Diaryl-niederalkylsilyl oder Triarylsilyl ist,

mit einer Verbindung der Formel

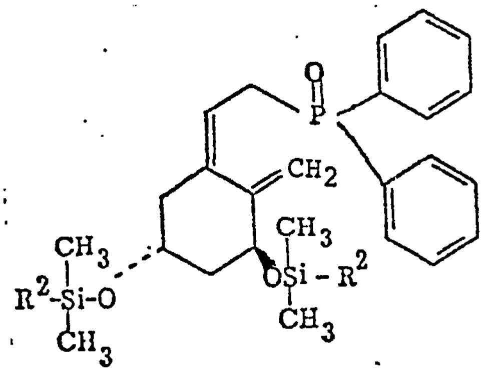

III

worin $R^2$ Niederalkyl oder Aryl ist, zu einer Verbindung der Formel

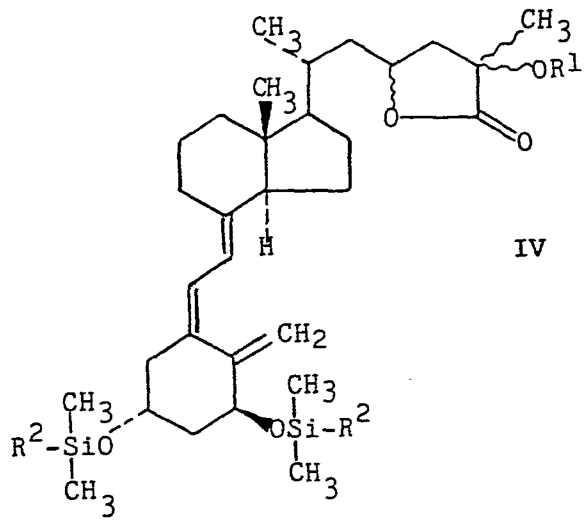

IV

worin $R^1$ und $R^2$ die obige Bedeutung haben, umsetzt und

b) die Schutzgruppen entfernt.

2. Verfahren nach Anspruch 1, zur Herstellung des 1α,23S,25R-Trihydroxy-cholecalciferol-26-carbonsäure-γ-lactons, dadurch gekennzeichnet, dass man von der entsprechenden Verbindung der Formel II ausgeht.

3. Eine Verbindung der Formel

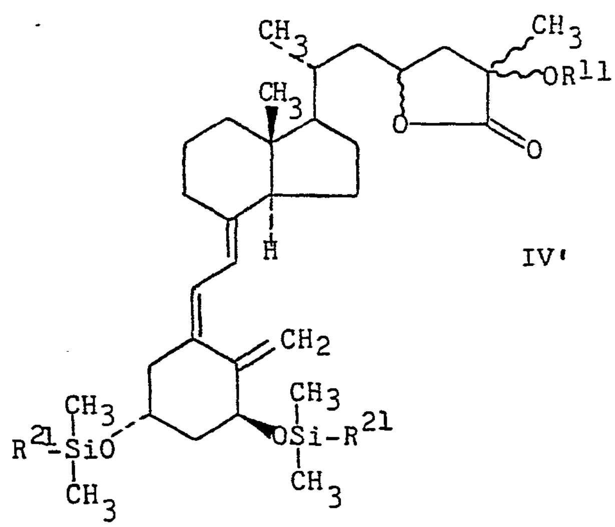

IV'

worin $R^{11}$ Tri-niederalkylsilyl oder Wasserstoff und $R^{21}$ Niederalkyl ist.

4. (1α,3β,5Z,7E,23R,25S)-1,3-Bis[dimethyl-(1,1-dimethyläthyl)silyloxy]-23-hydroxy-25-trimethylsilyloxy-9,10-secocholesta-5,7,10(19)-trien-26-carbonsäure-γ-lacton.

5. (1α,3β,5Z,7E,23S,25R)-1,3-Bis[dimethyl-(1,1-dimethyläthyl)silyloxy]-23-hydroxy-25-trimethylsilyloxy-9,10-secocholesta-5,7,10(19)-trien-26-carbonsäure-γ-lacton.

6. Eine Verbindung der Formel

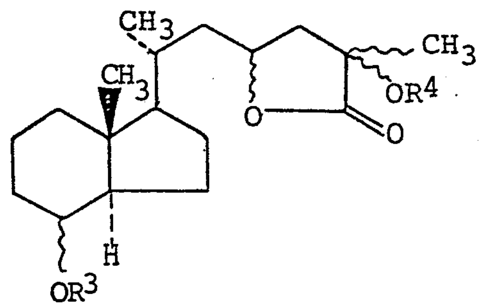

V

worin $R^3$ Wasserstoff, Tri-niederalkylsilyl, Aryl-diniederalkylsilyl, Diaryl-niederalkylsilyl oder Triarylsilyl und $R^4$ Wasserstoff, Tri-niederalkylsilyl, Aryl-di-niederalkylsilyl, Diaryl-niederalkylsilyl, Triarylsilyl oder eine Gruppe der Formel

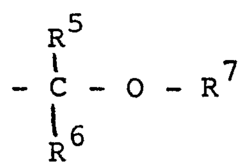

worin $R^5$ Wasserstoff oder Niederalkyl, $R^6$ und $R^7$ Niederalkyl oder Aryl, oder $R^6$ und $R^7$ zusammen $C_{3-6}$Alkylen sind.

7. Eine Verbindung nach Anspruch 6, worin $R^3$ Tri-niederalkylsilyl oder Wasserstoff und $R^4$ eine Gruppe der Formel

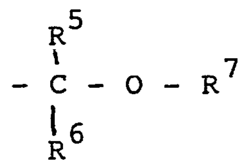

worin $R^5$ Wasserstoff und $R^6$ und $R^7$ Niederalkyl sind.

8. [3R-[3β,5S,5β(R*)-[1R*-(1β,3aα,4α,7aα)]]]-Dihydro-5-[2-(octahydro-4-trimethylsilyloxy-7a-methyl-1H-inden-1-yl)-2-methyläthyl]-3-(1-äthoxyäthoxy)-3-methyl-2(3H)-furanon.

9. [3S-[3α,5S,5β(R*)-[1R*-(1β,3aα,4α,7aα)]]]-Dihydro-5-[2-(octahydro-4-trimethylsilyloxy-7a-methyl-1H-inden-1-yl)-2-methyläthyl]-3-(1-äthoxyäthoxy)-methyl-2(3H)-furanon.

10. [3R-[3β,5S,5β(R*)-[1R*-(1β,3aα,4α,7aα)]]]-Dihydro-5-[2-(octahydro-4-hydroxy-7a-methyl-1H-inden-1-yl)-2-methyläthyl]-3-(1-äthoxyäthoxy)-3-methyl-2(3H)-furanon.

11. [3S-[3α,5R,5α(R*)-[1R*-(1β,3aα,4α,7aα)]]]-Dihydro-5-[2-(octahydro-4-hydroxy-7a-methyl-1H-inden-1-yl)-2-methyläthyl]-3-(1-äthoxyäthoxy)-3-methyl-2(3H)-furanon.

12. [3R-[3β,5S,5β(R*)-[1R*-(1β,3aα,4α,7aα)]]]-Dihydro-5-[2-(octahydro-4-hydroxy-7a-methyl-1H-inden-1-yl)-2-methyläthyl]-3-hydroxy-3-methyl-2(3H)-furanon.

13. [3S-[3α,5R,5α(R*)-[1R*-(1β,3aα,4α,7aα)]]]-Dihydro-5-[2-(octahydro-4-hydroxy-7a- methyl-1H-inden-1-yl)-2-methyläthyl]-3-hydroxy-3-methyl-2(3H)-furanon.

14. Eine Verbindung der Formel

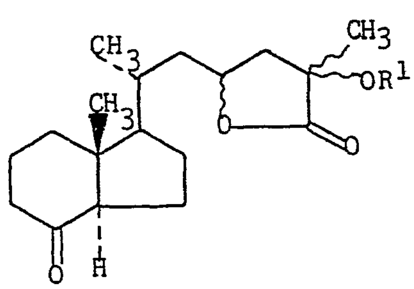

II

worin $R^1$ Wasserstoff, Tri-niederalkylsilyl, Aryl-diniederalkylsilyl, Diaryl-niederalkylsilyl oder Triarylsilyl ist.

15. [3R-[3β,5S,5β(R*)-[1R*-(1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon.

16. [3R-[3β,5R,5α(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon.

17. [3S-[3α,5R,5α(R*)-[1R*-[1β,3aα,4α,7aα)]]]-Dihydro-5-[2-(octahydro-7a-methyl-1H-inden-4-on-1-yl)-2-methyläthyl]-3-hydroxy-3-methyl-2(3H)-furanon.

18. [3S-[3α,5S,5β(R*)-[1R*-(1β,3aα,4α,7aα)]]]-Dihydro-5-[2-(octahydro-7a-methyl-1H-inden-4-on-1-yl)-2-methyläthyl]-3-trimethylsilyloxy-3-methyl-2(3H)-furanon.

***